(19)

Europäisches
Patentamt
European
Patent Office
Office européen
des brevets

(11)  **EP 2 229 142 B1**

(12)  **EUROPEAN PATENT SPECIFICATION**

(45) Date of publication and mention
of the grant of the patent:
**27.02.2019 Bulletin 2019/09**

(21) Application number: **08867815.6**

(22) Date of filing: **19.11.2008**

(51) Int Cl.:
*A61Q 1/02* *(2006.01)*        *A61K 8/02* *(2006.01)*
*A61K 8/04* *(2006.01)*        *A61K 8/19* *(2006.01)*
*A61K 8/25* *(2006.01)*        *A61K 8/26* *(2006.01)*
*A61K 8/29* *(2006.01)*        *A61K 8/73* *(2006.01)*
*A61K 8/81* *(2006.01)*        *A61K 8/88* *(2006.01)*
*A61K 8/891* *(2006.01)*       *A61K 8/894* *(2006.01)*
*B82Y 5/00* *(2011.01)*

(86) International application number:
**PCT/US2008/084017**

(87) International publication number:
**WO 2009/085444 (09.07.2009 Gazette 2009/28)**

(54) **GEL TECHNOLOGY SUITABLE FOR USE IN COSMETIC COMPOSITIONS**

GELTECHNOLOGIE FÜR KOSMETISCHE ZUSAMMENSETZUNGEN

TECHNOLOGIE DE GEL ADAPTÉE À UNE UTILISATION DANS DES COMPOSITIONS
COSMÉTIQUES

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **27.12.2007 US 16967 P**

(43) Date of publication of application:
**22.09.2010 Bulletin 2010/38**

(73) Proprietor: **Avon Products, Inc.
New York, NY 10017 (US)**

(72) Inventors:
• **MAITRA, Prithwiraj
Randolph
New Jersey 07869 (US)**
• **BROWN, Steven, E.
New Windsor
New York 12553 (US)**
• **GLYNN, John, R., Jr.
Ridgewood
New Jersey 07450 (US)**
• **ROTHOUSE, Jason
Mahwah
New Jersey 07430 (US)**

• **BRAHMS, John, C.
Morris Plains
New Jersey 07950 (US)**
• **FAIR, Michael, J.
Ridgewood
New Jersey 07450 (US)**

(74) Representative: **Dr. Weitzel & Partner
Patent- und Rechtsanwälte mbB
Friedenstrasse 10
89522 Heidenheim (DE)**

(56) References cited:
**EP-A1- 0 745 370        EP-A1- 1 386 600
EP-A1- 1 736 140        WO-A1-2009/075994
WO-A2-02/03935        US-A- 4 066 745
US-A1- 2004 170 586        US-A1- 2005 163 730
US-A1- 2005 163 813        US-A1- 2007 190 011
US-A1- 2007 196 299        US-B2- 6 593 395
US-B2- 7 205 340**

• **DATABASE WPI Week 199644 Thomson
Scientific, London, GB; AN 1996-439436
XP002742313, & JP H08 217637 A (KAO CORP)
27 August 1996 (1996-08-27)**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

**FIELD OF THE INVENTION**

The present invention relates to gel technology comprising macroscopic particles dispersed/suspended/interspersed within a network of fractal particles, more particularly to cosmetic compositions containing such gels to obtain efficient optical blurring of wrinkles, fine lines, pores, skin imperfections, and the like, and most particularly to such cosmetic compositions in which the fractal network is a fractal gel.

**BACKGROUND OF THE INVENTION**

A number of methods have been developed to reduce wrinkles and minimize fine lines. Some of these methods include active ingredients such as antioxidants; agents that act by neurotransmission inhibition in nerve cells such as botulinum toxin (Botox™) (Allergan, Irvine, Calif.), thereby relaxing contracted muscles, agents that accelerate the cell renewal process such as hydroxy and fruit acids like retinoic acid; emollients such as shea butter; skin plumpers such as hyaluronic acid; fillers such as collagen; light-diffusing pigments and microspheres which create the illusion that wrinkles have disappeared. Other methods have been developed to reduce the appearance of pores, skin surface unevenness and imperfections. Some of these methods include skin lightening agents, filling and camouflaging the skin.
Unfortunately, many cosmetic foundations and make-ups actually accentuate wrinkles and fine lines due to migration of the pigments into the wrinkle crevices. Other products cover skin imperfections but create an unnatural, caked-on appearance. Others, such as mica, reflect rather than diffuse and scatter light, thereby resulting in an unnatural shiny appearance. Additionally, some of these methods are not immediate, requiring days and weeks of continued use to see effects. Others are invasive, requiring injections, patent discomfort, and may entail redness, swelling and other side effects.
Novel, safe and effective topical "optical blurring" technology to treat wrinkles and skin imperfections are needed. Therefore, the need exists for alternative methods to provide a natural and smooth appearance to the skin with visible reduction in wrinkles, fine lines, pores and skin imperfections but which overcomes the problems associated with previous methods and compositions and which would represent a significant advance in cosmetic art.
The optical reduction of wrinkles is due to the light diffusing properties of the applied particles At the margins and in the creases of wrinkles, particles that scatter and thus diffuse light away minimize the depressions in the skin. To the observer, the wrinkles appear blurred, hence the terms "soft focus effect" "blurring effect." In the past, the blurring effect was based on the diffuse reflection of spherical particles such as microspheres and fibers. One such composition is that described by Nakamura, N, et al, "Blurring of Wrinkles Through Control of Optical Properties". XIVth I.F.S.C.C. Congress. Barcelona, Spain, 1986.
The incorporation of inorganic nanoscale particles into a polymeric matrix is known for industrial uses to provide clear coatings, for example, mobile phones or skies.

**SUMMARY OF THE INVENTION**

The present invention provides for the use of fractal particles with unique optical properties and surface chemistry, combined with micron dimension organic or inorganic particles such that the fractal network wrap around the macroscopic particles increasing the interfacial area over which lateral light diffusion occurs. It has been found that such technology is useful to optimize the optical diffusion effect of light, *i.e.*, optical blurring, and consequently, cause the appearance of wrinkles, fine lines, pores and skin imperfections to vanish while allowing the skin to appear natural and flawless.
The present application describes a gel system (as hereinafter further described) comprising macroparticles within a fractal network of nanoparticles.
The present application further describes such gel system comprising macroparticles that are translucent, namely silicone crosspolymers.
The present application further describes the gel system in which the fractal network is a fractal gel (as hereinafter further described).
The present application yet describes cosmetic compositions comprising the gels of the present invention that are efficient in blurring fine lines, wrinkles, pores, and skin imperfections.
The present application also describes gels that leverage the differences in size domain and optical properties between fractal particles and macroscopic particles. The presence of macroscopic particles increase the spatial distribution of fractal particles increasing the interfacial area over which light bending/lateral scattering occurs. Accordingly, the gels are seen to have superior optical properties when used especially in cosmetic products. Macroscopic particles are selected from silicone cross-polymers.

The present application further describes cosmetic compositions containing aqueous gels according to the invention comprising macroscopic particles present in a fractal particle network obtained by using a mixture of fractal particles with opposite zeta potential at a given pH. Such aqueous gels may be used as prepared, may be modified to include other ingredients, or may be incorporated as a phase in an emulsion cosmetic composition.

The present application further describes cosmetic compositions containing anhydrous gels of the invention comprising macroscopic particles dispersed in a network of fractal particles, typically with a compatible anhydrous solvent. Such anhydrous gels may be used as prepared, may be modified to include other ingredients, or may be incorporated as a phase in an emulsion cosmetic composition.

The present application also describes methods for producing the gels of the invention and cosmetic compositions containing same.

The present application also describes a cosmetic treatment process allowing wrinkles, fine lines, pores and skin imperfections to be blurred in human beings, particularly the skin of the face, neck, and lips, this process being characterized by applying an effective quantity of a composition described in the present application to the skin.

It is an object of the present invention, to provide methods for blurring wrinkles and fine lines. A method includes applying to the skin and/or lips a gel composition which leverages the relative size/domains and refractive indices of the fractal network and macroscopic particles to obtain efficient blurring.

In another aspect of the invention the present invention is applicable to the skin in a cosmetically acceptable vehicle.

The above objects will be achieved by the features of claim 1.

## BRIEF DESCRIPTION OF THE FIGURES

FIG. 1 is a schematic representation of the spatial arrangement of the gel structure comprising the macroparticles and the fractal nanoparticles.

FIG. 2 is a schematic representation illustrating the diffusion on light incident on the surface of skin treated with a cosmetic composition of the present invention.

FIG. 3 is a graphical plot of the zeta potential of various metal oxides as a function of pH.

## DETAILED DESCRIPTION OF THE INVENTION

The present invention utilizes gel systems to provide compositions having unique optical and space filling properties. As a consequence to the optical properties, thin films of the compositions applied to a substrate, in particular, a biologic surface, change the way light incident on the surface of the film is refracted and improves the diffusion of incident light on the surface of the film. When the composition is a cosmetic composition and the film is on the surface of the skin of an individual, the imperfections of the skin are less noticeable, *i.e.*, less "visible" because of the way reflected light is being seen by an observer. In addition the gels described in the present application provide a fractal network with macroscopic particles, thus further providing beneficial optical properties, especially when the refractive indices are non-matching. The gels are capable of filling voids in the surface of the biologic substrate, especially, wrinkles, line, pores, and other imperfections present in the surface of an individual's skin or lips. Unless indicated otherwise, the term "gel" refers to the gels as set forth in this paragraph and as further described in this specification.

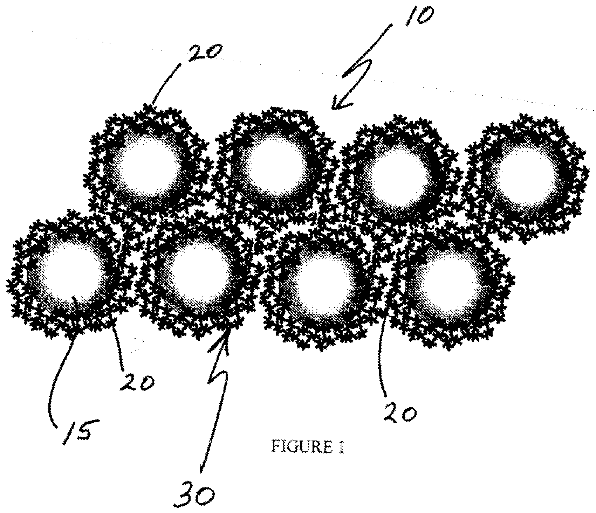

As shown schematically in FIG. 1, it is believed that gel 10 comprises a plurality of translucent macroparticles 15 surrounded by a multiplicity of nanoparticles 20, which nanoparticles aggregate or otherwise coalesce to form a fractal gel network, represented generally by numeral 30.

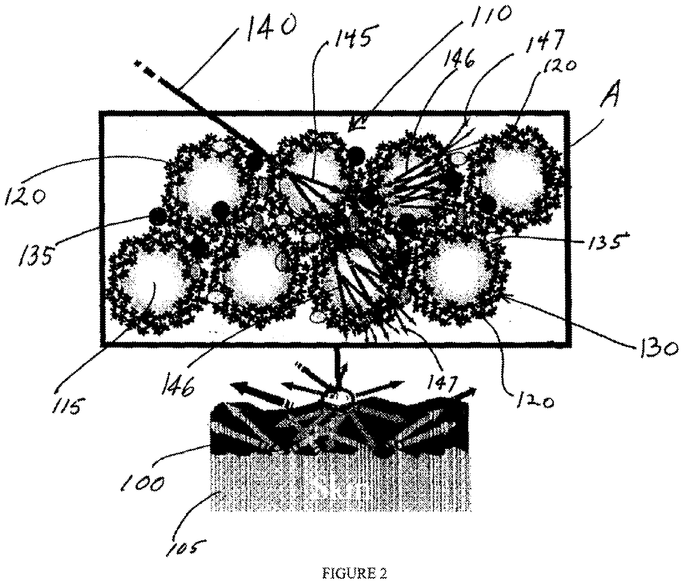

FIG. 2 illustrates a film 100 of a cosmetic composition applied to skin 105, as well as an enlarged view A of the gel 110 taken from a small area of the film 100. Gel 110 comprises a plurality of translucent macroparticles 115 surrounded by a multiplicity of nanoparticles 120, whereby fractal gel network 130 is formed. Actives and/or adjuvants 135 are present within the gel network 130. Light 140 entering the gel 110 is diffused by the translucent macroparticles, as shown schematically by the plurality of light vectors 145, 146, and 147, whereby the skin is provided with an optical blurring benefit

Another beneficial aspect of the invention is the ability of the gel network to display unique rheological properties, which are especially useful in cosmetic applications. The gel network is highly thixotropic. That is to say, the viscosity of the gel rapidly diminishes under increasing shear stress, yet the gel network reforms quickly once the shear stress is removed. Effectively, this imparts an effect wherein the composition transforms from viscous, non-flowing compositions to a free flowing liquid when the composition is applied, e.g., with a brush or other applicator. The speed at which the network reforms to a gel is a function of particle concentration, and, in the instance where the fractal network is a fractal gel, on the magnitude of the attractive interaction between the oppositely charged particles (refer to section "Surface Charge of Particulate Dispersions"). Hyperthixotropic compositions are particularly useful in foundations, mascaras, hair care, lip compositions, and personal care compositions where low viscosity is desired during application, yet a rapid increase in viscosity is important to prevent migration of the applied composition.

The gel system comprises one or more translucent macroparticles and includes a fractal network of nanoparticles. Translucent materials allow light to pass through them but scatter light so that the material distorts the image. Suitably translucent macroparticles are those whose diffuse transmittance is greater than zero for a 10 micron film cast on a glass plate as measured using a color(i) spectrophotometer. Films can be prepared by dispersing macroparticles in a suitable binder, polymer, or solvent. A dispersion can prepared by dispersing macroparticles in a binder, polymer or solvent followed by casting a 10 micron film on a glass (normalized with % solids in the solution) using a drawdown bar. A color (i) spectrophotometer can be used to measure total transmittance and direct transmittance. Diffused transmittance can be obtained by subtracting direct transmittance from total transmittance.

In the present invention the fractal network comprises a first and second fractal particles and the fractal network is a fractal gel. While not wishing to be bound by any particular theory or mechanism, the fractal network is believed to envelop the macroparticles, with gelation occurring when dispersed in a suitable medium.

The phrase "cosmetically acceptable vehicle" refers to a medium that is compatible with keratin materials, such as human skin.

The term "optical blurring" as used herein refers to optical reduction of wrinkles, fine lines, skin surface unevenness and imperfections.

The term "macroscopic particles" or "macroparticles" as used herein refers to particles that have a size range of 1 to 200 microns.

The term "nanoparticle" refers to particles with a size of up to about 900 nm.

The term "fractal particles" as used herein refers to nanoparticles of varying fractal dimension or in-built reticulated structure; that is, having Hausdorff-Besicovitch dimensions greater than their topological dimensions. As used herein, "nanoparticles" is synonymous with "fractal particles", unless specifically indicated otherwise.

By "gel" is meant any co-continuous phases of macroscopic particles and a fractal particle network that forms a composite gel structure.

The terms "blurring" and "optical blurring" as used herein refers to optical reduction of wrinkles, fine lines, pores and skin surface unevenness and imperfections.

Reference to "particle size" means the mean diameter of particles measured under an appropriate imaging technique for the size domain under consideration, for example, scanning electron microscopy or transmission electron microscopy.

Except where specific examples of actual measured values are presented, numerical values referred to herein should be considered to be qualified

The terms "a" and "an", as used herein and in the appended claims, mean "one or more" unless otherwise indicated herein.

All percentages and ratios referred to herein are by weight of total composition (*i.e.*, the sum of all components present), unless otherwise indicated.

The Fractal Network

The fractal network comprises a first and second type of submicron sized fractal particle (i.e., nanoparticles). When dispersed in a suitable medium the fractal particles coalesce to form the fractal network. Depending on the medium, the coalescing takes place in light of van der Waals forces (hydrophilic dispersant), electrostatic attraction, or because of hydrogen bonding (lipophilic dispersant). As explained in more detail below, the macroparticles may be added to this dispersion under shear to form the gels of the present invention.

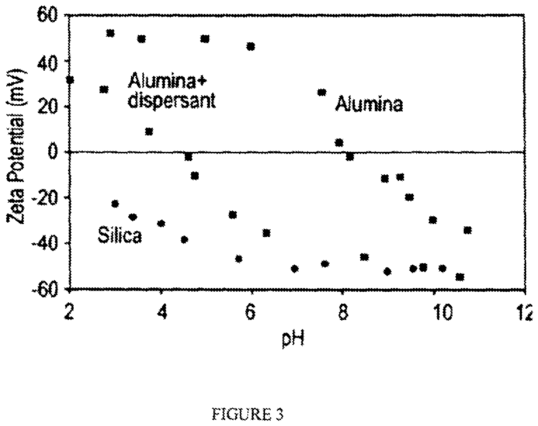

The fractal network is a fractal gel. The fractal gel comprises first and second submicron sized fractal particles having opposite surface charges at a given pH. By way of example and referring to FIG. 3, at pH below 7-8 the metal oxides silica and alumina have opposite surface charge or zeta potential. The first or second fractal particles that form the fractal gel may each comprise two or more different fractal particles having the same charge. The two or more different first or second fractal particles of the same charge may have different sizes, different net surface charges (of the same type, however), or different refractive indices. The fractal gels, because of their oppositely charged particles, have stronger attraction between the fractal particles.

The fractal gel is obtainable by forming dispersions of the oppositely charged fractal particles. Combining aqueous dispersions of each particle type forms a highly structured gel network as a result of charge neutralization. Alternatively, the zeta potentials may be of the same sign initially, and the pH of the combined dispersions adjusted thereafter to give zeta potentials of opposite signs, thereby allowing integration of the organic particles and inorganic particles.

A brief description of fractal particle geometry follows:

Fractal objects are characterized by a recursive self-similarity. In general, the fractal nature can be described mathematically by a power law relationship taking the form:

$$Y = c * X^d \qquad (1)$$

where c is a constant. Therefore, if data adhere to a power law relationship, a plot of log (Y) versus log (X) will yield a straight line with slope d.

Analogously, self-similar fractals, a class of Hausdorff-Besicovitch dimensionality, rely on the object being self-similar at different length scales. The power law is consistent with this case following:

$$A = (1/s)^D \qquad (2)$$

where A is the number of identical parts, $s$ is the reduction factor and D is the self-similar dimension measure of the fractal. Equation 2 can be arranged as the following

$$D = \log (A)/\text{Log } (1/S) \qquad (3)$$

For example, the sides of a unit square are divided in half, forming 4 pieces, therefore A = 4, s = ½ thus D equals 2. Likewise a Sierpinski Gasket, wherein the original triangle side is halved, three triangle pieces are formed. Thus, A = 3. s = ½ and D = 1.5850. Comparatively, consider a unit line segment. Dividing the line in half results in 2 equal parts, and so on. Therefore, A =2, s = ½ D = 1. It is important to note, the value of D agrees with the topological dimension of the line, yet a line is not fractal. Accordingly, fractals are those objects wherein the Hausdorff-Besicovitch dimension exceeds its topological dimension.

Furthermore, fractals can be classified according to their self-similarity. There are three basic types of self-similarity expressed in fractals. Exact self-similarity (the strongest type of self-similarity). The fractal appears identical at different length scales. Fractals of this type are described by displaying exact self-similarity.

Quasi-self-similarity (non-exact form of self-similarity). The fractal appears approximately identical at different length scales. Quasi-self-similar fractals are comprised of distorted and degenerate copies.

Statistical self-similarity (weakest type of self-similatity). The fractal is described by statistical measures, which are preserved across the length scale. Random fractals are examples of fractals, which are statistically self-similar, but not exact or quasi self-similar. The nature of similarity of fractals can also be described by mathematical functions.

Most fractal objects of interest do not have a readily apparent self-similar nature. Therefore, a convenient method to determine the fractal dimension of the object is the box counting method. This method is widely used and a direct method to measure the fractal dimension objects via image analysis. An object image is projected on a grid of known dimensions Subsequently, the number of blocks that the image touches is counted. This data yields the number of blocks (N) and the block size (reduction factor, s). The grid is resized, and the process is repeated. A plot of the data, where the x-axis is log (s) and the y-axis is log (N(s)) using equation 3, yields a slope of value D.

Image analysis is particularly useful to evaluate the fractal dimension of particulates. Specifically, transmission electron spectroscopy (TEM) is well adapted to evaluate the fractal dimension of complex particulate structures. Of particular interest are fractal particles that are comprised of non-overlapping primary particles, which form a larger aggregate structure. Typically, particles of this nature are manufactured by a fuming process or complex precipitation process,

Evaluation of the mass fractal dimension of particles formed from aggregates of smaller primary particles involves determination of the number of primary particles per aggregate. Typically, this is achieved by evaluating TEM micrographs using digital imaging processing techniques. The number of primary particles per aggregate (N) is determined by dividing the projected area of the aggregate (Aa) by the projected area of the monomer unit (Am):

$$N = (Aa/Am)^{\alpha} \qquad (4)$$

where $\alpha$ is an empirical fitting parameter, typically 1.0-1.1. Therefore, the Hausdorff dimension implies the relationship between the primary particle size (dp), the area radius of gyration (Rg), and the number of primary particles (N) describes the fractal dimension (Df) of the aggregate:

$$N = kf \, (Rg/dp)^{Df} \qquad\qquad\qquad (5)$$

where kf is a constant fractal prefactor. A plot of log (N) vs. log (Rg) results in a linear plot of slope Df. Typical Df values for fractal particles of the present invention obtained by a fuming process range from 1.5-1.9, while fractal particles of the present invention obtained by a precipitation process range from 2-2.8.

Additional test methods based on rheological measurements and light scattering measurements can be used to elucidate the dimensionality of fractal particles, as known in the art.

The fractal nature of the particles results in a porous matrix structure of the fractal network. In another embodiment the porous matrix structure of the fractal network may receive one or more active substances.

The size domains and refractive indices of the fractal particles are chosen to effectively form a barrier between the macroscopic particles and consequently enhance the ability of the compositions according to the specification to fill wrinkles and mask skin imperfections. The fractal particle network has an open structure, which provides a surface smoothing effect. Thus, the composition fills in imperfections in the surface of the skin, and thus provides a natural, smooth and youthful appearance with visible reduction in wrinkles and skin imperfections when applied to the skin.

Typically, the fractal particle may comprise between about 5% to about 75%, preferably about 10-40%, most preferably about 20-40% solid fractal particles by weight of the fractal particle dispersion. In some instances the particles are provided by the manufacturer as a dispersion. Suitable commercially available metal oxide dispersions are Cab-o-Sperse™ PG01, PG063, PG003, PG0042, and AeroDisp™ W1836, W630 supplied by Cabot Corporation and Degussa, respectively.

Any suitable metal oxide fractal particles or derivatives thereof that achieve result in a reticulated fractal network may be employed. Preferably, the inorganic nanoparticles particles are fractal metal oxide particles having a diameter of between about 25-300 nm, preferably about 40-250 nm, and more preferably about 40-200 nm. Diameter as used herein refers to the diameter of a sphere that encompasses the fractal particle. Diameter may be determined by methods known in the art, e.g., light scattering and TEM. Furthermore, each nanoparticle type has a particle surface area between about 50 to 400 m$^2$/g, and more particularly between about 50 to 300 m$^2$/g, The fractal dimension of the nanoparticle ranges from about 1.2 to 2.8, preferably from about 1.5 to 2.5. Generally, as fractal dimension increases, the concentration of solids in the network1 decreases, and as surface area increases, fractal dimension also increases.

While not common, fractal organic particles are known and can be used in accordance with the present invention, provided the requisite surface charge characteristics are met. For example, organic polyacrylates and their derivatives have fractal dimensionality and may be surface charged. Preferred organic polyacrylate particles are lauryl methacrylate/dimethyl acrylate crosspolymer (available from Amcol Health and Beauty Solutions).

The fractal particles may be selected from the group consisting of silica, alumina, titania, zirconia, zinc oxide, indium tin oxide, ceria, and mixtures thereof. Particles may be formed as part of a fuming process or a precipitation process wherein the metal oxide particle is fractal in dimension. Particles formed by the fuming process are preferred. Alumina is known to impart high diffuse transmittance, high reflectance, high scattered reflectance and low total reflectance in the visual spectra, and is a preferred fractal particle. Silica is preferred because it has a refractive index that is substantially matchable to common cosmetic media, especially silicone oils.

In the fractal network in the form of a fractal gel, alumina and silica are preferred oppositely charged particles. As shown in FIG. 3, silica is available with a net surface charge that is opposite to that of alumina at a pH value of most cosmetic formulations, that is, at a pH below about 7 - 8. Accordingly, silica is a preferred second fractal particle, especially when used in conjunction with alumina at a composition pH less than about 7 to 8.

Examples of suitable fractal particles include, but are not limited to, fumed silicas sold by Degussa under the tradename Aerosil, including hydrophilic and hydrophobic fumed silicas, for example, the Aerosil R-900 series, A380™ fumed silica (manufactured by Degussa), OX50™ (manufactured by Degussa), colloidal silica such as the Cabosil™ line (manufactured by Cabot), fumed alumina such as SpectrAL™ (manufactured by Cabot), and fumed titania. Preferred is fumed silica, fumed alumina, fumed titania (Degussa W740X) fumed zirconia (Degussa W2650X, W255OX), fumed ceria (Degussa Adnano) fumed zinc oxide (Degussa Adnano), fumed indium tin oxide (Degussa Adnano) or mixtures thereof.

Charged particles are subject to electrophoresis, that is to say, in the presence of an electric field they move with respect to the liquid medium in which they are dispersed. The region between the particle and the liquid is known as the plane of shear. The electric potential at the plane of shear is called the zeta potential. The magnitude and sign of this potential can be experimentally determined using commercially available equipment. Typically, to achieve colloidal stability, i.e. prevent floccuiaiion, charged particulates are required to have a minimum zeta potential of approximately 25 mV.

Selection of fractal particle pairs for the fractal gel can be chosen based on the magnitude and sign (positive or negative) of the zeta potential at a given pH. Preferably, the magnitude and sign of the zeta potential of each particle type is sufficient, such that when combined, a non-settling semi-rigid gel structure is formed. Preferred dispersions of the first particle type have a zeta potential values of about +10 mV to +50mV, more preferably +10mV to + 30mV. and most preferably + 15mV to + 25mV. Preferred dispersions of the second particle type have a zeta potential values of about -10 mV to -50mV, more preferably -10mV to -30mV, and most preferably -15mV to -25mV. Furthermore, evaluation of the point of zero charge (isoelectric point) of metal oxides is useful to pre-select metal oxides of interest, as listed in Table 1.

Surface Chargeof Particulate Dispersions

The presence of charge on dispersed colloidal particles occurs by two principal mechanisms: dissociations of ionogenic surface groups or preferential absorption. Each mechanism can occur simultaneously or independently. Dissociation of acidic groups on the surface of a particle will give rise to a negatively charged surface. Conversely, dissociation of basic surface groups will result in a positively charged surface, In both cases, the magnitude of the surface charge depends on the strength of the acidic or basic groups and on the pH of the solution. The surface charge can be reduced to zero (isoclectric point) by suppressing the surface ionization. This can be achieved by decreasing the pH in the case of negatively charged particles or increased the pH in the case of positively charged particles, Furthermore, if alkali is added to a dispersion of negatively charged particles, the particles tend to become more negatively charged, If acid is added to this dispersion, then a point will be reached where the charge on the particle is neutralized. Subsequent addition of acid will cause a build up of positive charge on the particle.

Modification of Surface Charge

Adsorption of ions and ionic surfactants can be specifically adsorbed onto the charged particle surface. In the case of cationic surfactants, adsorption leads to a positively charged surface and in the case of anionic surfactants, adsorption leads to a negatively charged surface. Adsorption of single valent or multivalent inorganic ions (*e.g.* $Na^+$, $Al^{+3}$) can interact with charged surfaces in one of two ways: reduction of the magnitude of charge at a given pH: change in pH of the isoelectric point (point of neutral charge). The specific adsorption of ions onto a particle surface, even at low concentrations, can have a dramatic effect on the surface charge. In some cases, specific ion adsorption can lead to a charge reversal of the surface. The addition of surfactants or specific ions to particle dispersions is a common method to modify the surface charge characteristics.

Table 1. Point of Zero Charge (PZC) for Various Oxides in Water

| Oxide | PZC | Oxide | PZC | Oxide | PZC |
|---|---|---|---|---|---|
| $Ag_2O$ | 11.2 | HgO | 7.3 | SnO2 | 5.6 |
| $Al_2O_3$ | 9.1 | $La_2O_3$ | 10.1 | $Ta_2O_5$ | 2.8 |
| BeO | 10.2 | MgO | 12.4 | $ThO_2$ | 9.2 |
| CdO | 11.6 | $MnO_2$ | 5.3 | $TiO_2$ Rutile | 5.7 |
| $CeO_2$ | 8.1 | $MoO_3$ | 2 | $TiO_2$ Anatase | 6.2 |
| CoO | 10.2 | $Nb_2O_5$ | 2.8 | $V_2O_3$ | 8.4 |
| $Co_3O_4$ | 7.4 | NiO | 10.2 | $WO_3$ | 0.4 |
| $Cr_2O_3$ | 7.1 | $PuO_2$ | 5.3 | $Y_2O_3$ | 8 9 |
| CuO | 9.3 | $RuO_2$ | 9 | ZnO | 9.2 |
| $Fe_2O_3$ | 8.2 | $Sb_2O_5$ | 1.9 | $ZrO_2$ | 7 6 |
| $Fe_3O_4$ | 6.6 | $SiO_2$ | 2 | | |

The Macroscopic Particle

The macroscopic particles used in the preparation of the gels of the present application are translucent, and are within the matrix of the gel. However, it is understood that the cosmetic composition may be multiphasic, for example, an emulsion wherein the gel is dispersed into a continuous external phase. Alternatively, additional components may be dispersed into the gel phase. In yet another embodiment, macroscopic particles may have a charge or surface functionality which increases interaction with the fractal particles.

In yet another embodiment, two different fractal particles are used to form different fractal networks, which are used to form, along with macroscopic particles, the gels of the application. In another embodiment different macroscopic particles can be used to form the gel, using the same or different fractal networks.

Preferably, the refractive index of the fractal particle does not match the refractive index of the macroparticle. Non-match means as used in this context that the refractive index values are about 0.05 or more units from one another, preferably more than about 0.07, and most preferably more than about 0,1.

The macroscopic particles of the invention have a particle size of between about 1-200 microns, preferably between about 2-100 microns. Macroscopic particles are silicone crosspolymers. In another preferred embodiment the macroscopic particles are silicone crosspolymers having a particle size of from about 1 to about 200 microns.

Such panicles are prepared by conventional procedures, for example, by palletizing, cutting, or tearing a bale of the elastomeric material into shreds or small pieces followed by chopping or grinding those shreds or small pieces into particles having the size desired. In addition "wet" chemistry techniques known in the art may be used to form particles of a particular size or distribution of particle sizes that are desirable. The practice of the present invention does not depend on the particular procedure utilized to prepare the elastomer and elastomeric particles.

Suitable macroscopic particles useful in the invention especially for skin care applications have a refractive index from 1,38 to 1.6.

Suitable are silicone crosspolymers obtained by self polymerization of bifunctional precursor molecules containing both epoxy-silicone and silyl hydride functionalities to provide a silicone copolymer network in the absence of crosslinker molecules. Such crosspolymers are described in US Patents 6,444,745; 6,531,540; 6,538,061; 6,759,479, and in US Appln. 2003/00959935. Especially suitable are such crosspolymers such as the Velvesil™ line of silicone crosspolymers available from Momentive Performance Materials, Inc. (formerly GE Silicones). Most preferred is Velvesil 125™ (GE), a cyclopentasiloxane and C30-C45 alkyl cetearyl dimethicone crosspolymer.

The weight ratio of the fractal particles to macroscopic particles in the gels of the present invention are typically from about 1:10 to 10:1, preferably from about 1:10 to 2:1, and most preferably from about 1:5 to 1:1.

Compositions

The cosmetic composition may take on various forms including powder, cake, pencil, stick, ointment, cream, milk, lotion, liquid-phase, gel, emulsion, emulsified gel, mousse, foam, spray, wipes. Preferably, the cosmetic composition is used in a liquid or powder foundation.

The gels may be incorporated in cosmetically acceptable vehicles, such as but not limited to, liquid (*e.g.,* suspension or solution), gel, emulsion, emulsified gel, mousse, cream, ointment, paste, serum, milk, foam, balm, aerosol, liposomes, solid (*e.g.,* pressed powders), anhydrous oil and wax composition. Preferably, the cosmetic composition is used in a liquid or powder foundation. More specifically, the cosmetic include facial skin care cosmetics such as skin lotion, skin milk, skin cream, gel, and make-ups such as foundation, foundation primer base, blush, lip stick, eye shadow, eye liner, nail enamel, concealer, mascara, body make-up product, or a sunscreen.

A person skilled in the art can select the appropriate presentation form, and also the method of preparing it, on the basis of general knowledge, taking into account the nature of the constituents used and the intended use of the composition.

Cosmetic compositions according to the application may comprise from about 1 to 100% gel, and typically comprise from about 2 to about 90%,preferably comprise 10 to 80% and most preferably comprise 30 to 55% gel by weight of the cosmetic composition. The broad range reflects the range of different types of cosmetic products and the various product forms; namely, gels, emulsions, and dispersions. Typically, the gel contains about 3% to about 60% fractal particles by weight of the gel, and more typically from about 5% to about 40% fractal particles by weight of the gel. Amounts of the gel in the cosmetic compositions of the application are also discussed later. Useful gel compositions may include alumina and silica, titania and silica, zirconia and silica, and other combinations of particulates described within.

The cosmetic compositions of the present application may be formulated as aqueous or nonaqueous compositions, which may be emulsions or non-emulsions. In one embodiment, the cosmetic compositions according to the application are formulated as emulsions. These emulsions may be oil-in-water (including silicone in water) emulsions, water-in-oil (including water-in-silicone) emulsions, or multiple emulsions such as oil-in-water-in-oil (o/w/o) or water-in-oil-in-water (w/o/w), but are preferably silicone-in-water emulsions, It is understood that the oil phase can comprise silicone oils, non-silicone organic oils, or mixtures thereof. While not preferred, the compositions can comprise two immiscible phases that are admixed at the time of use by shaking.

In a typical embodiment in which a gel having a fractal gel is employed, the weight ratio of alumina to silica is 1 :1 to 9:1 and is present as a dispersion in water wherein the alumina surface area is between 50 to 200 m2 g and the silica surface area is between about 50 to 400 m2/g. Suitable gels can be formed by using Spectral 51 or Spectral 80 (Cabot Corporation) fumed alumina and Cab-O-Sil M5, Cab-O-Sil EH-5. Furthermore, dispersions of metal oxides can be chosen based on their surface charge characteristics as determined by zeta potential measurements. Generally, particle size, surface area and surface charge determines the ease with which the gel forms and its physical attributes such as yield strength.

In addition to the gel phase comprising the fractal particles and the macroparticles, the compositions of the present application may comprise one or more active ingredients adapted to bestow a cosmetic benefit to the skin when applied to the skin as a film and/or one or more adjuvants or excipients (adjuvants and excipients are collectively referred to herein as adjuvants) to impart to the cosmetic product particular desirable physical properties, to meet product performance requirements, or to establish compositional type, e.g., emulsion (of a particular type), solution. The actives and/or the adjuvants may be present in the gel phase including the fractal network or the fractal gel, as the case may be, in another phase, or in either, as desired, or as mandated by the chemical system.

Suitable active agents include pigments to impart a color to the skin or other biologic surface; opacifiers and light diffusers; sunscreens; uv light absorbers; emollients; humectants; occlusive agents; antioxidants; exfoliants; antioxidants; anti-inflammatory agents; skin whitening agents; abrasives; antiacne agents; hair treatment agents; humectants; emollients; moisturizers; anti-wrinkle ingredients; concealers; matte finishing agents; proteins; anti-oxidants; bronzers; solvents; ultraviolet (UVA and/or UVB) absorbing agents; oil absorbing agents; neutralizing agents. It is understood to those skilled in the art that any other cosmetically acceptable ingredient, *i.e.*, those included in the International Cosmetic Ingredient Dictionary and Handbook, 11th Edition (2006) (Cosmetic and Toiletries Association) (hereinafter identified as INCI) may be used and compatible combinations thereof.

Suitable adjuvants include film forming agents; solvents; viscosity and rheology modifiers such as thickeners; surface active agents including emulsifiers; hydrotropes: emulsion stabilizers; plastieizers; fillers and bulking agents; pH adjusting agents including buffers, acids, and bases; chelating agents; binders; propellants: fragrances; preservatives and antimicrobials, and compatible combinations thereof

Suitable active agents and adjuvants used in cosmetic compositions of the present invention are tabulated in INCL Generally, reference to specific materials utilizes the INCI adopted name nomenclature. The active agents and adjuvants are incorporated in the compositions of the present application in amounts that provide their intended functions, as those skilled in the cosmetic arts are knowledgeable. Generally, this amount is from about 0.001 to 25%, more usually 0.01 to 15%, and especially 0.1 to 10% by weight of the composition.

The cosmetic compositions may contain polymeric light diffusers such as nylon (*e.g.*, Nylon 12 available from Cabot as SP-500 and Orgasol 2002™), cellulose beads, poly(methylacrylic acid) (also known as PMMA or methyl methacrylate crosspolymer; CAS No. 25777-71-3), polyethylene, polystyrene, ethylenc/acrylic acid copolymer (e.g., EA-209 supplied by Kobo), and fluorinated hydrocarbons such as Teflon The polymeric light diffusers, preferably nylon, are present in a concentration in the range of between about 0.01-10%, preferably about 0.1-5% by weight of the composition. Inorganic light diffusers can also be used, e.g., boron nitride, barium sulfate, and silicates such as calcium alumina borosilicate, and are typically present in an amount of from about 0.01 to about 10%, preferably about 0.1 to about 5% by weight.

The cosmetic composition of the present application may contain a viscosity modifier such as a thickener together with emulsifiers to modify the viscosity of the composition, for example to form creams, pastes, and lotions that enhance skin feel. Suitable viscosity modifiers are starches, cellulose derivatives such as sodium carboxymethyl cellulose, methyl cellulose, ethyl cellulose, cationized cellulose, hydroxyethyl cellulose, hydroxypropyl cellulose, hydroxypropylmethyl cellulose; silicates such as Veegum or clays; polysaccharides such as xanthan or guar gums, hydrophilic polymers, such as carboxyvinyl polymers, for example carbomers. Viscosity/rheology modifiers may be present in the composition in an amount of from about 0.1 to about 10% by weight of the composition.

The cosmetic emulsifier should preferably be an oil-in-water or water-in-oil emulsifier. Preferably, the oil phase is a silicone oil, and the emulsifier is a silicone emulsifier. The emulsifiers may be chosen advantageously to match the refractive index of the fractal particle whose refractive index is matched, but are not substitutes for the refractive index matching polymer.

Emulsifying agents may be present in a concentration of from about 0- 10%, preferably about 0,1-6%, more preferably about 3-5%. Nonlimiting examples of suitable emulsifiers are glycerol monostearate, PEG 12 Dimethicone (Dow Corning), RM 2-2051™ (Dow Corning), an emulsion of aqueous polyacrylate emulsified into silicone (dimethicone and cyclopentasiloxane), alkylmethyl siloxanes copolyol (Dow Corning 5200), PEG 11 methylether dimethicone (Shin Etsu), cyclopentasiloxane/PEG/PPG 18/18 dimethicone (Dow Corning 5225C).

The cosmetic composition of the present application may contain non-occlusive film-forming agents such as, but not limited to, cosmetic fluids, i.e., silicone compounds containing various combinations of elastomers in a variety of diluents. Examples of suitable cosmetic fluids are cyclopentasiloxane and amino propyldimethicone (Cosmetic fluid 1486-NH) (manufactured by Chemisil), cyclomethicone and dimethicone (Cosmetic Fluid 1684-DM) (manufactured by Chemisil), and a blend of low and high viscosity polydimethylsiloxane (e.g. Dow Corning 1413 Fluid™) (Dow Corning). Preferred is a blend of high viscosity polydimethylsiloxane in low viscosity polydimethylsiloxane (e.g. Dow Corning 1413 Fluid™) (Dow Corning).

In one embodiment the cosmetic composition is nonpigmented.

In one embodiment the cosmetic compositions contain one or more pigments, which are typically present in a different phase from the gel phase. The pigment used herein can be inorganic and/or organic. Cosmetic compositions according to the application comprise greater than or equal to 0.1% pigments by weight of the cosmetic composition to provide a pigmenting effect. Typically, the pigments may be present from about 0.1% to 25%, preferably from about 0,5 to 15%, and most preferably from about 1 to 10% by weight The pigments are not fractal particles in accordance with the invention because they do not have the proper size domain, do not have the proper dimensionality, or are not charged particles. As used herein the term "pigments" includes lakes, and a single pigment or pigment combinations. Other colorants such as D&C dyes and self-tanning agents such as carbonyl derivatives or food colorants such as dihydroxyacetone (DHA) or erythrulose may be used. Pigments and colorants are used interchangeably herein.

Preferably, the pigments are selected from the group consisting of titanium oxides such as rutile titanium dioxide, anatase titanium dioxide, zinc oxide, zirconium oxide, iron oxides such as ferric oxide, ferrous oxide, yellow iron oxide, red iron oxide, bismuth oxy chlorides, black iron oxide, acylglutamate iron oxides, chromium oxide, chromium hydroxide, manganese violet, cerium oxide, ultramarine blue, carmine, and derivatives and mixtures thereof. More preferably, the pigment is titanium oxide, yellow iron oxide, red iron oxide, black iron oxide, and mixtures thereof. The pigments can be surface modified to render them either hydrophobic or hydrophilic to interact synergistically with the fractal particle network.

The cosmetic composition may also include opacifying agents (pearlescent agents) to add optical shimmer and luster or for tactile silkiness to the touch such as, but not limited to mica, sericite (a fine grained variety of muscovite). These agents may be present in amounts from about 0.1-10%, preferably about 0.5-5%.

The cosmetic composition may also include oil phase solvents useful as base fluids for spreading and lubrication properties or as a vehicle to provide a medium for one or more of the other constituents of the cosmetic composition. Solvents include water, organic fluids, especially alcohols and hydrocarbon fluids, silicone fluids, hydrophilic and hydrophobic polymers, and may be present in a concentration of about 0.5-90%, preferably about 5-50%, most preferably 10-35%. Preferred oil phase solvents are cyclomethicones such as cyclotetrasiloxane (*e.g.* Cyclo-2244 Cosmetic Grade Silicone (D4) (manufactured by Clearco), cyclopentasiloxane (*e.g.* Cyclo-2245 Cosmetic Grade Silicone (D5) (manufactured by Clearco), a cyclopentasiloxane/cyclohexasiloxane blend (D5/D6 Blend) Cyclo-2345 Cosmetic Grade Silicone (manufactured by Clearco), and a cyclomethicone/dimethiconol blend (D5/D4 Blend) Cyclo-1400 Cosmetic Grade Silicone (manufactured by Clearco). More preferred is D5.

Water typically is present in amounts ranging from about 10% to about 95% water by weight of the composition, preferably from about 40% to about 80%, and most preferably from about 40% to about 70%. Also suitable as aqueous phase solvents are low molecular weight alcohols having less than 8 carbons, for example ethanol, propanoic hexanol, and the like, and polyhydric alcohols, especially glycols. Suitable glycols are propylene glycol, pentylene glycol, hexylene glycol, and 1,2-octanediol. Suitable polyhydric alcohols include sorbitol and glycerin. These may be present in amounts of from about 1% to about 50 %, preferably 5% to 35% by weight.

Optionally, electrolytes such as sodium chloride may be added in amounts ranging from about 0-5%, preferably from about 0.5-2%.

The compositions of the application further typically contain an amount of a pH adjusting agent to provide the desired pH of the composition, e.g., the pH at which the fractal particles will have the requisite opposite net surface charges Suitable pH adjusting agents are organic and mineral acids as is well known in the cosmetic arts. Buffers to maintain the established pH may also be incorporated, for example sodium lactate.

It is further understood that the other cosmetic actives and adjuvants introduced into the composition must be of a kind and quantity that are not detrimental to the advantageous effect which is sought herein according to the invention.

The composition of the present application improves the optical properties of films of cosmetic composition (as compared to those which merely reflect light producing a shiny appearance, those which merely cover the skin and impart a white cast to the skin, or those which either result in optical blurring or space filling, but not both). The resulting composition when applied to the skin, makes the skin appear more youthful, smoother and even in tone,

The physical arrangement of the gel structure, high particle loading and network formation, provides a smooth surface for topcoat (optical layer) applications of any foundation. The optical layer provides a unique "light releasing" effect from the skin when used as a primer for pigmented cosmetics. The optical layer mimics and enhances the skin's natural transparent qualities. When light penetrates the optical layer, diffuse reflection through the pigmented layer provides a "back lighting" effect, brightening foundations to give a more natural and youthful look

Methods of Use

The methods of use for the cosmetic compositions disclosed herein concern the improvement in the aesthetic appearance of skin and include, but are not limited to: methods of blurring or masking one or more of wrinkles, fine lines, pores, skin imperfections, especially in the facial, neck or on or around the lip areas; methods to correct imperfections in skin such as blotches, freckles, redness, spider veins, and dark rings around the eyes; methods of enhancing or modifying skin color; and methods to improve finished makeup, and methods for application to the hair, eyelashes, and eyebrows.

The compositions of the present application are suitable for use as a hair cosmetic. in particular as a mascara, in light of the unique rheological properties exhibited by the gels, as mentioned above. Thus, the compositions of the application are free-flowing under shear, which allows them to be applied with a brush or suitable applicator. When the shear is removed the compositions return rapidly to the more viscous gel condition. Because the compositions are fractal, that is, they are porous, reticulated structures capable of maintaining geometric shape, they are able to coat hair and provide a volumizing benefit. Accordingly, they are ideal as mascaras, especially when formulated with a film former (as previously described), and as hair volumizers for treating thinning hair.

Examples of facial lines and skin imperfections which can be improved using the fractal gels of the present application include, but are not limited to; frown lines that run between the eyebrows known as glabellar lines; perioral or smoker's lines which are vertical lines on the mouth; marionette lines at the corner of the mouth known as oral commissures; worry lines that run across the forehead; crow's feet at the corner of the eyes known as periorbital lines; deep smile lines that run from the side of the nose to corners of the mouth known as nasolabial furrows; cheek depressions; acne scars; some facial scars; wound or burn scars; keloids, to reduce dark rings around the eyes; to reduce the appearance of pores or blemishes, age spots, moles, birthmarks; to redefine the lip border; for artificial or self-tanning, and to reduce skin color unevenness or dullness.

In another embodiment the resulting gel can be employed as it is and can itself constitute a skin care or make-up composition for blurring wrinkles, fine lines, pores, and skin imperfections.

Therefore, the gels may comprise from about 1% to about 100% by weight, relative to the total weight of the composition

Facial lines and wrinkles can be present anywhere on the face, and occur most frequently on the lips and in the eye area. However, it is understood by those skilled in the art that the composition can be applied to any part of the body where a blurring effect is desired such as to reduce wrinkles, fine lines, poses and skin imperfections. Non-limiting examples include to conceal imperfections in the skin, such as to mask the appearance of cellulite or vitiligo, to mask the appearance of spider vessels, moles, age spots, blemishes, scars, freckles, birth marks and varicose veins, to conceal damage incurred to the skin as a result of trauma such as cosmetic surgery, burns, stretching of skin, to conceal the appearance of villus hair on the skin; to provide UV protection to the skin.

The compositions herein can be used by topically applying to the areas of the skin an effective amount of the compositions. The effective amount can easily be determined by each user. As used herein the term "effective amount" refers to an amount sufficient to result in "optical blurring" of the appearance of the skin.

The composition can be applied for several days, weeks, months or years at any intervals. The compositions are generally applied by light massaging the composition onto the skin. However, the method of application may be any method known in the art and is thus not limited to the aforementioned.

It is further understood that the gel of the present application may be used together with therapeutic agents together with or adjunctive to pharmaceutical compositions including, but not limited to, anti-acne agents, sunscreens, self-tanning ingredients, anti-inflammatory agents, anti-bacterials, anti-fungals, anti-virals, anti-yeast agents, eye treatments, age spot treatments, analgesics, antidandruff and antiseborrhetic agents, hyperkeratolytics, antipsoriatic agents, skin lightening agents, depigmenting agents, wound healing agents, burn treatments, tanning agents, hair treatment agents, hair growth products, wart removers, antipuretics, and hormones.

Preparation

Sol-gel chemistry techniques can be used to effect the formation of the gels of the present application, as described in C.J. Brinker and W Scherer, Acad. Press, Boston, 1990.

The compositions useful for the methods of the present invention are generally prepared by conventional methods such as are known in the art of making topical cosmetic compositions. Such methods typically involve mixing of the ingredients in one or more steps to a relatively uniform state, with or without heating, cooling, application of vacuum.

Typically, the network of fractal particles is made by preparing a dispersion of each fractal particle in a suitable solvent (dispersant), adjusting the dispersion pH with a pH adjusting agent, if needed, e.g., when the fractal network is a fractal gel, and admixing the dispersions with shear to permit the formation of the fractal network. In some instances owing to the properties of the constituents it may be necessary to preheat the dispersant. Where fractal gels are being used to form the fractal network, the pH adjusting agent may also be provided into the admixed dispersions rather than into each dispersion individually. The macroparticles may then be incorporated into the dispersion, along with any actives and adjuvants that are desired. Certain of the adjuvants may require addition as premixes with a solvent, as generally known in the cosmetic art. The resulting gel can be employed as it is and can itself constitute a skin care or make-up composition for blurring wrinkles and skin imperfections.

Alternatively, the fractal gel may be incorporated into a multiphase cosmetic composition as previously mentioned. The other phase may be prepared in accordance with known methods, for example forming one or more premixes of the ingredients for combination with the gel. As previously mentioned the polymer in whole or in part may be incorporated into this other phase. Where premixes have been formed at elevated temperatures appropriate cooling of the composition to establish the emulsion will be necessary.

The following examples describe specific aspects of the invention to illustrate the invention and provide a description of the present methods for those skilled in the art.

## EXAMPLES

General Example

The present indention is further illustrated by the following nonlimiting example.

Table 1: Illustrative Compositions

| Component | WEIGHT % |
|---|---|
| Polydimethylsioxane and Cetearyl Dimethicone Crosspolymer[1] | 5-50 |
| Cellulose Beads (spherical) | 1-20 |
| Fumed Alumina (SpectrAL. 51 from Cabot) | 0.1-10 |
| Amino Phenyltrimethicone | 0.1-10 |
| Ethylhexylmethoxy Cinnamate | 0.1-7.5 |
| Acrylates/Dimethicone copolymer-cyclomethicone | 0.1-10.0 |
| Decamethyl cyclopentasiloxane | 0.1-30 |
| Fragrance | 0-1 |
| Fumed Silica (Cabosil EH-5 from Cabot) | 0.1-10 |
| Preservative | 0.1-2 |
| Nylon Powder (spherical) | 0.1-10 |
| HDI/ Trimethylol Hexyllactone C rosspoly mer and Silica[2] | 0.1-10 |
| Polyethylene 1-20 um[3] (spherical) | 0.1-10 |
| Boron Nitride (spherical) | 0.1-10 |
| Ethylhexyl Salicylate | 0.1-5 |
| POE (24M) Cholesterol Ether [4] | 0.1-5 |
| Lauryl PEG-9 Polydimethylsiloxane Dimethicone [5] | 0.1-5 |

[1]Available from Dow Corning under the tradename DC 9041.
[2]Available from Kobo Products under the tradename BPD 800
[3]Available from Presperse under the tradename Micropoly 220L.
[4]Available from Croda under the tradename Crodalan C24.
[5]Available from Shin Etsu under the tradename KF6038.

In the method of making of the preferred compositions of the present invention, the gel emulsifying agent, and sunscreens are premixed in a vessel. To a separate vessel, the solvent, film formers, wax, and preservative are added and heated to 82.2 to 87.8 °C (180 to 190 degrees F). with mixing. Once the temperature is constant and the materials are well mixed, the fumed alumina and silica are added. Mixing continues until all of the fumed material is evenly dispersed. The premixed gel phase is then added to the solvent/film former wax mixture. Mixing continues for 10 to 60 minutes, as the batch cools the remaining powdered components are added. Fragrance is added when the temperature is below 48.9 °C (120 °F).

When wax is used as a structurant in the composition the processing temperature is maintained above the solidification point of the wax and a hot fill is used.

Table 2: illustrative Composition Containing Wax

| Component | WEIGHT % |
|---|---|
| Polydimethylsiloxane and Cetearyl Dimethicone Crosspolymer[1] | 5-50 |
| Cellulose Beads (spherical) | 1-20 |
| Fumed Alumina (SpectfAL 51 from Cabot) | 0.1-10 |
| Ethylhexylmethoxy Cinnamate | 01-7.5 |
| Acrylates Dimethicone copolymer/cyclomethicone | 0.5-10.0 |
| Decamethyl cyclopentasiloxane | 0.1-30 |
| Fragrance | 0-1 |
| Fumed Silica (Cabosil EH-5 from Cabot) | 0.1-10 |
| Preservative | 0.1-2 |
| Nylon Powder (spherical) | 0.1-10 |
| HDI/Trimethylol Hexyllactone Crosspolymer and Silica[2] | 0.1-10 |
| Polyethylene 1-20 um[3] (spherical) | 0.1-10 |
| Boron Nitride (spherical) | 0.1-10 |
| Ethylhexyl Salicylate | 0.1-5 |
| C30-45 Alkyl Methicone/C30-45 Olefin | 0.1-5 |
| Lauryl PEG-9 Polydimethylsiloxane Dimethicone[5] | 0.1-5 |
| [1] Available from Dow Corning under the tradename DC 9041.<br>[2] Available from Kobo Products under the tradename BPD 800.<br>[3] Available from Presperse under the tradename Micropoly 220L.<br>[5] Available from Shin Etsu under the tradename KF6038. | |

Skin care compositions of the present invention are found to reduce the visibility of wrinkles to a greater extent than skin care products not containing the gels of the present invention.

Working Examples (without Pigment)

Examples I through VI are illustrative of the present invention in which Examples I - III are aqueous emulsions and Examples IV - VI are anhydrous compositions, wherein the fractal particles 3, 4 and 5 are dispersed in D5.

Table 3: Colorless Primer Compositions

|  |  |  | I | II | III | IV | V | VI |
|---|---|---|---|---|---|---|---|---|
|  | 1 | Dow Corning 1413 Fluid | 10 | 10 | 10 | 15 | 15 | 15 |
|  | 2 | Velvesil 125 | 30 | 30 | 30 | 35 | 30 | 30 |
|  | 3 | Fumed Silica | 0 | 0 | 0 | 7 | 10 | 7 |
|  | 4 | Fumed TiO2 | 0 | 0 | 0 | 0 | 5 | 8 |
|  | 5 | Fumed Alumina | 0 | 0 | 0 | 10 | 5 | 5 |
|  | 6 | Nylon | 0 | 0 | 0 | 3 | 5 | 5 |
|  | 7 | Dow Corning 5225 C | 0 | 0 | 9 | 0 | 0 | 0 |
|  | 8 | D5 | 15 | 15 | 9 | 30 | 30 | 30 |
|  | 9 | RM 2-2051 (Dow Corning) | 4 | 4 | 0 | 0 | 0 | 0 |
|  | 10 | Water | 11 | 11 | 11 | 0 | 0 | 0 |

13

(continued)

| | | I | II | III | IV | V | VI |
|---|---|---|---|---|---|---|---|
| 11 | 30% Silica/Alumina (1:1) dispersion in Water | 0 | 30 | 15 | 0 | 0 | 0 |
| 12 | 30% Silica/Alumina (2:1) dispersion in Water | 30 | 0 | 15 | 0 | 0 | 0 |
| 13 | NaCl | 0 | 0 | 1 | 0 | 0 | 0 |
| | Total | 100 | 100 | 100 | 100 | 100 | 100 |

1 Dimethicone (3300 cs.) sold by Dow Corning Corp. as DC 1413 fluid
2 Macroscopic dimethicone/vinyl dimethicone crosspolymer available from Momentive Performance Materials, Inc.
3 Cabosil EH-5 available from Cabot Company
4 Aeroxide P25 from Degassa
5 SpectrAl 51 from Cabot
6 Orgasol 2002 B Natural Extra COS from Lipa Chemical
7 Cyclopentasiloxane and PEG/PPG-18/18 Dimethicone sold by Dow Corning Corp.
8 Cyclomethicone available from Dow Corning under the tradename DC 245 Fluid
9 Thickening agent containing sodium polyacrylate, dimethicone, cyclopentasiloxane, trideceth-6 and PEG/PPG-18/18 dimethicone sold by Dow Corning Corp.
11 and 12 are fractal particle gel dispersions containing fumed silica (Cabosil EH-5) and fumed alumina (SpectrAl 51) both sold by Cabot Corporation in the proportions indicated

The compositions I through III were made as follows:

Phase A - Silicone Phase: Dow Corning 1413 Fluid. Velvesil 125, DC 5225 C were mixed until homogeneous followed by addition of D5 and Nylon using a 3 blade propeller mixer. RM 2051 was then added to the silicone phase and mixed for an additional 10 minutes.

Phase B - Aqueous phase: A 30 wt% dispersion of fumed silica/alumina was added to water and NaCl until homogenous for about 20 minutes at room temperature. Phase B was then slowly added to phase A with continuous stirring for 10 minutes. The composition was then mixed further for an additional 20 minutes.

The compositions IV through VI were made as follows:

Velvesil 125, D5, and (1413 Fluid are added and heated to 82.2 to 87.8 °C (180 to 190 degrees F) with mixing. Once the temperature is constant and the materials are well mixed, the fumed alumina and silica are added. Mixing continues until all of the famed material is evenly dispersed. Mixing continues for 10 to 60 minutes, as the batch cools the remaining powdered components (nylon) are added.

Working Examples with Pigment

The following examples are illustrative of a foundation composition containing pigments.

Table 4: Foundation Compositions

| | | I | II | III | IV | V |
|---|---|---|---|---|---|---|
| 1 | TiO2 | 5 | 4 | 3 | 5 | 5 |
| 2 | Red Iron Oxide | 2 | 3 | 2 | 2 | 3 |
| 3 | Yellow Iron Oxide | 3 | 2 | 2 | 3 | 2 |
| 4 | Black Iron Oxide | 1 | 1 | 1 | 1 | 1 |
| 5 | Sericite | 5 | 4 | 5 | 3 | 3 |
| 6 | Dow Corning 1413 Fluid | 10 | 10 | 10 | 10 | 12 |
| 7 | Velvesil 125 | 25 | 25 | 25 | 30 | 27 |
| 8 | Fumed Silica | 0 | 0 | 0 | 8 | 7 |
| 9 | Fumed TiO2 | 0 | 0 | 0 | 5 | 8 |

(continued)

| | | | I | II | III | IV | V |
|---|---|---|---|---|---|---|---|
| 10 | Fumed Alumina | | 0 | 0 | 0 | 5 | 2 |
| 11 | Nylon | | 0 | 0 | 0 | 5 | 5 |
| 12 | Dow Corning 5225C | | 0 | 0 | 10 | 0 | 0 |
| 13 | D5 | | 10 | 10 | 9 | 23 | 25 |
| 14 | Rm 2-2051 (Dow Corning) | | 4 | 4 | 0 | 0 | 0 |
| 15 | Water | | 5 | 7 | 8 | 0 | 0 |
| 16 | 30% SilicalAlumina (1:1) dispersion in Water | | 0 | 30 | 12 | 0 | 0 |
| 17 | 30% Silica/Alumina (2:1) dispersion in Water | | 30 | 0 | 12 | 0 | 0 |
| 18 | NaCl | | 0 | 0 | 1 | 0 | 0 |
| | Total | | 100 | 100 | 100 | 100 | 100 |

6 Dimethicone (3300 cs.) sold by Dow Corning Corp. 1431 Fluid is the trade name 7 Macroscopic dimethicone/vinyl dimethicone crosspolymer available from Momentive Performance Materials, Inc.

8 Cabosil EH-5 available from Cabot Company 9 Aeroxide P25 from Degussa 10 SpectrAL 51 from Cabot 11 Orgasol 2002 B Natural Extra COS from Lipa Chemical 12 Cyclopentasioxane and PEG/PPG-18/18 Dimethicone sold by Dow Corning Corp.

13 Cyclomethicone available from Dow Corning under the tradename DC 245 Fluid 14 Thickening agent containing sodium polyacrylate, Dimethicone, Cyclopentasiloxane, Trideceth-6 and PEG/PPG-18/18 Dimethicone sold by Dow Corning Corp.

16 and 17 are fractal particle gel dispersions containing fumed silica (Cabosil EH-5) and fumed alumina (SpectrAl 51) both sold by Cabot Corporation in the proportions indicated

Examples I - III are aqueous emulsions; Examples IV - V are anhydrous compositions in which the fractal particles 8. 9 and 10 are dispersed in D5.

The present invention provides a variety of compositions useful in solid and/or semi-solid forms (including creams, gels and viscous liquids). Such compositions are preferably foundations, but also include face sticks, pancakes, and other facial cosmetic products.

**Claims**

1. A method for optically blurring the appearance of skin imperfections selected from the group consisting of wrinkles, fine lines, and pores comprising the step of applying to the skin an amount of a skin care or make-up composition effective to optically blur the appearance of said skin imperfections, wherein the skin care or make-up composition comprises a gel comprising: (a) a fractal gel network of nanoparticles; and (b) translucent macroscopic particles of a silicone cross-polymer enveloped in said fractal network of nanoparticles; wherein the nanoparticles are inorganic nanoparticles having a particle size of between 50 to 900 nm and a refractive index of from 1.38 to 2; and wherein the macroscopic particles have a refractive index of from 1.38 to 1.6; and wherein the gel is an aqueous gel and the fractal particle network is obtained by forming an aqueous dispersion of first and second nanoparticles with oppositely charged zeta potentials at a given pH; and wherein the oppositely charged nanoparticles coalesce to form the fractal network.

2. The method of claim 1 wherein the first nanoparticles are alumina and the second nanoparticles are silica.

3. The method of claim 1, wherein the translucent macroscopic elastomeric particles comprise cyclopentasiloxane and C30-45 alkyl cetearyl dimethicone crosspolymer.

**Patentansprüche**

1. Verfahren zur optischen Verwischung des Erscheinungsbildes von Hautunvollkommenheiten, ausgewählt aus der Gruppe, die aus Falten, feinen Linien und Poren besteht, umfassend den Schritt des Auftragens einer Menge an Hautpflege- oder Make-up-Zusammensetzung auf die Haut, die wirksam ist, um das Erscheinungsbild der Hautunvollkommenheiten optisch zu verwischen, wobei die Hautpflege- oder Make-up-Zusammensetzung ein Gel umfasst, umfassend: (a) ein fraktales Gelnetz aus Nanopartikeln; und (b) lichtdurchlässige makroskopische Partikel eines Silikon-Kreuzpolymers, die in das fraktale Netz aus Nanopartikeln eingehüllt sind; wobei die Nanopartikel anorganische Nanopartikel sind, die eine Partikelgröße zwischen 50 bis 900 nm und einen Brechungsindex von 1,38 bis 2 aufweisen; und wobei die makroskopischen Partikel einen Brechungsindex von 1,38 bis 1,6 aufweisen; und wobei das Gel ein wässriges Gel ist und das fraktale Partikelnetz durch Bilden einer wässrigen Dispergierung von ersten und zweiten Nanopartikeln mit entgegengesetzt geladenen Zetapotentialen bei einem gegebenen pH erhalten wird; und wobei die entgegengesetzt geladenen Nanopartikel verschmelzen, um das fraktale Netz zu bilden.

2. Verfahren nach Anspruch 1, wobei die ersten Nanopartikel Aluminiumoxid sind und die zweiten Nanopartikel Siliziumdioxid sind.

3. Verfahren nach Anspruch 1, wobei die lichtdurchlässigen makroskopischen elastomeren Partikel Cyclopentasiloxan und C30-45-Alkyl-Cetearyl-Dimethicon-Kreuzpolymer umfassen.

**Revendications**

1. Procédé pour estomper optiquement l'apparence d'imperfections de la peau choisies dans le groupe consistant en les rides, les ridules et les pores comprenant les étapes d'application à la peau d'une quantité d'une composition de soin ou de maquillage de la peau efficace pour estomper optiquement l'apparence desdites imperfections de la peau, dans lequel la composition de soin ou de maquillage de la peau comprend un gel comprenant : (a) un réseau de gel fractal de nanoparticules ; et (b) des particules macroscopiques translucides d'un polymère réticulé de silicone enveloppées dans ledit réseau fractal de nanoparticules ; dans lequel les nanoparticules sont des nanoparticules inorganiques ayant une taille de particule entre 50 à 900 nm et un indice de réfraction entre 1,38 et 2 ; et dans lequel les particules macroscopiques ont un indice de réfraction entre 1,38 et 1,6 ; et dans lequel le gel est un gel aqueux et le réseau fractal de particules est obtenu en formant une dispersion aqueuse de premières et secondes nanoparticules avec des potentiels zêta de charge opposée à un pH donné ; et dans lequel les nanoparticules de charge opposée coalescent pour former le réseau fractal.

2. Procédé selon la revendication 1, dans lequel les premières nanoparticules sont en alumine et les secondes nanoparticules sont en silice.

3. Procédé selon la revendication 1, dans lequel les particules élastomériques macroscopiques translucides comprennent du cyclopentasiloxane et un polymère réticulé de diméthicone d'alkyle en C30 à 45-cétéaryle.

FIGURE 1

FIGURE 2

FIGURE 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6444745 B
- US 6531540 B
- US 6538061 B
- US 6759479 B
- US 200300959935 A

**Non-patent literature cited in the description**

- **NAKAMURA, N et al.** Blurring of Wrinkles Through Control of Optical Properties. *XIVth I.F.S.C.C. Congress,* 1986
- International Cosmetic Ingredient Dictionary and Handbook. Cosmetic and Toiletries Association, 2006
- *CHEMICAL ABSTRACTS,* 25777-71-3